# FASCICULE DE BREVET EUROPEEN

(11) **EP 3 416 576 B1**
(45) Date de publication et mention de la délivrance du brevet: **19.07.2023**
(21) Numéro de dépôt: 17710326.4
(22) Date de dépôt: 15.02.2017
(51) Int. Cl.: A61B 17/70, A61B 17/82, A61B 17/84, A61B 17/88

(54) **IMPLANT INTERVERTÉBRAL DE STABILISATION DYNAMIQUE ET KIT CHIRURGICAL L'INCORPORANT**
DYNAMISCH STABILISIERENDES WIRBELSÄULENIMPLANTAT UND CHIRURGISCHES KIT DAMIT
DYNAMICALLY STABILIZING VERTEBRAL IMPLANT, AND SURGICAL KIT COMPRISING SAME

(30) Priorité: 15.02.2016 FR 1651203
(43) Date de publication de la demande: 26.12.2018
(73) Titulaire: Backbone, 33110 Le Bouscat (FR)
(72) Inventeur: SENEGAS, Jacques, 33700 Mérignac (FR)
(74) Mandataire: Plasseraud IP
(86) Numéro de dépôt international: PCT/FR2017/050346
(87) Numéro de publication internationale: WO 2017/140984

(56) Documents cités:
- EP-A1- 2 351 534
- WO-A1-2009/141393

## Description

### Domaine Technique

La présente invention se rapporte de manière générale aux implants utilisés dans la chirurgie du rachis, et plus particulièrement à un implant intervertébral de stabilisation dynamique ainsi qu'à un kit chirurgical l'incorporant.

### Arrière-plan Technologique

Les opérations dans le domaine de la chirurgie du rachis peuvent concerner la région cervicale (nuque), la région dorsale ou, plus fréquemment, la région lombaire.

Lorsqu'il existe une instabilité, comme par exemple un glissement d'une vertèbre par rapport aux vertèbres adjacentes, une opération de stabilisation de la colonne vertébrale peut consister à implanter du matériel métallique sous forme de vis reliées entre elles par des barres ou des plaques. Ces implants constituent un échafaudage qui joue un rôle de stabilisateur de la colonne vertébrale.

Selon des techniques plus récentes, on peut obtenir la stabilisation de la colonne grâce à un implant intervertébral composé d'une cale de stabilisation, d'un lien souple de type tresse textile, d'un ensemble mobile et d'un organe de verrouillage. La cale de stabilisation est destinée à prendre place entre les apophyses épineuses de deux vertèbres consécutives, c'est-à-dire adjacentes, à stabiliser. Le lien souple (par exemple une tresse textile) enserre les apophyses. L'ensemble mobile est adapté pour venir en engagement avec la cale de stabilisation de manière à bloquer le lien souple en mouvement par rapport à la cale de stabilisation. Ce blocage est obtenu par pincement du lien souple entre l'ensemble mobile et la cale de stabilisation. L'organe de verrouillage (par exemple une vis) est adapté pour verrouiller l'engagement de l'ensemble mobile avec la cale de stabilisation, et ainsi le blocage final du lien souple qui en résulte directement.

### Art Antérieur

Un implant intervertébral du genre précité est divulgué, par exemple, dans le document EP 2192863 publié par l'OMPI sous le numéro de publication WO 2009/040380, et dans le document EP 2303163 publié par l'OMPI sous le numéro de publication WO 2009/141393.

La plupart des opérations lombaires sont effectuées en foyer ouvert par l'arrière (voie d'abord postérieure), en pratiquant une incision dans le dos du patient au niveau des vertèbres à stabiliser.

La conception des implants de l'art antérieur implique la réalisation de gestes par le chirurgien qui nécessitent de dégager une zone d'intervention assez large autour des vertèbres à stabiliser, notamment pour la mise en place du ou des liens souples dans la cale, et pour la mise en tension et le blocage de ce ou de ces liens.

Le document EP 2138122 décrit un système de stabilisation entre le sacrum et une vertèbre lombaire. Le système comprend au moins une tresse textile qui est ancrée sur le sacrum par des vis. Pour assurer le blocage en tension des tresses un bloqueur comprend des pions coinceurs, entre les parois internes d'un orifice prévu dans le corps du bloqueur. La direction de déplacement d'un tel pion mobile est tangente à la surface de la tresse et est parallèle à l'axe longitudinal de ladite tresse. Ces dispositions ne sont pas adaptées à un implant intervertébral comme celui qui fait l'objet de la présente invention.

### Résumé de l'invention

Il importe de procurer au chirurgien un implant intervertébral de stabilisation dynamique qui permette de réduire la taille de l'incision au strict minimum, pour protéger les tissus environnants (notamment les tissus musculaires qui participent à la stabilité de la colonne vertébrale) du stress lié à l'écartement de la plaie chirurgicale qui peut entrainer des nécroses sévères. La récupération du patient suite à l'opération chirurgicale en est d'autant plus rapide, et avec un résultat d'autant plus satisfaisant.

A cet effet, un premier aspect de l'invention propose un implant intervertébral de stabilisation dynamique comprenant :
- une cale de stabilisation adaptée pour stabiliser entre elles au moins deux vertèbres adjacentes par interposition entre des apophyses épineuses des vertèbres, ayant un corps sensiblement parallélépipédique qui présente un axe principal déterminé,
- au moins une sangle formant lien souple pour la fixation de la cale de stabilisation aux apophyses épineuses des vertèbres à stabiliser, ladite sangle comportant une première et une seconde portions de sangle comprenant chacune l'une des extrémités opposées de la sangle, et ladite sangle présentant une direction longitudinale déterminée et une surface de sangle déterminée,
- au moins un ensemble mobile adapté pour venir en engagement avec la cale de stabilisation, de manière à bloquer la sangle en mouvement par rapport à la cale de stabilisation par pincement de la sangle entre l'ensemble mobile et la cale de stabilisation, et
- un organe de verrouillage pour verrouiller en position axiale l'engagement de l'ensemble mobile avec la cale de stabilisation et ainsi le blocage de la sangle.

Selon des modes de réalisation, le corps de la cale de stabilisation comprend au moins un évidement dans lequel peut passer au moins la première portion de sangle, l'évidement ayant un axe longitudinal parallèle à l'axe principal de la cale de stabilisation, et une paroi interne s'étendant parallèlement audit axe longitudinal de l'évidement avec une forme déterminée;
- l'ensemble mobile comprend au moins un pion de blocage ayant un axe longitudinal déterminé et une forme déterminée sensiblement complémentaire de la forme de l'évidement, pour venir en engagement avec la cale de stabilisation par déplacement suivant la direction de l'axe principal du corps de ladite cale à l'intérieur de l'évidement de telle manière que l'axe longitudinal du pion de blocage :
- premièrement coïncide alors avec l'axe longitudinal de l'évidement ;
- deuxièmement est perpendiculaire à l'axe longitudinal de la première portion de sangle à l'intérieur de l'évidement ; et,
- troisièmement est parallèle à la surface de la première portion de sangle à l'intérieur de l'évidement,
et de telle manière, en outre, que la portion de sangle à l'intérieur de l'évidement est bloquée en mouvement par rapport à la cale de stabilisation par pincement de ladite portion de sangle formant lien souple entre le pion de blocage et la paroi interne de l'évidement.

L'axe principal ci-dessus coïncide avec l'axe de la voie d'abord postérieure pour l'opération chirurgicale consistant à mettre l'implant en place. Le logement prévu dans la cale de stabilisation étant ouvert vers l'arrière lorsque la cale est mise en position d'installation, son axe longitudinal correspond à l'axe de la voie d'abord postérieure (par opposition, par exemple, à un accès latéral, ces termes « postérieure » ou « latérale » étant ceux du vocabulaire utilisé dans ce domaine de la chirurgie du rachis). Le pion de blocage peut ainsi commodément et utilement être inséré en force dans le logement suivant l'axe de la voie d'abord postérieure. La zone d'intervention et d'insertion (foyer ouvert) peut donc être réduite au strict minimum. L'invention autorise ainsi un mode opératoire réellement mini-invasif et un traitement chirurgical court de type ambulatoire.

Dans un mode de réalisation directement complémentaire il peut être prévu, en outre, que :
- chacune des première et seconde portions de sangle peut passer dans le logement prévu dans la cale de stabilisation, de manière pour la sangle à former au moins une boucle dans un plan perpendiculaire à l'axe principal de la cale, avec une ganse adaptée pour venir en engagement avec l'une des apophyses épineuses de deux vertèbres à stabiliser, et de manière que, en outre,
- le pion de blocage peut venir en engagement avec la cale de stabilisation suivant la direction de l'axe principal du corps de ladite cale entre chacune des première et seconde portions de sangle (41a,42a) à l'intérieur de l'évidement, de telle manière que l'axe longitudinal du pion :
   - premièrement coïncide alors avec l'axe longitudinal de l'évidement ;
   - deuxièmement soit perpendiculaire à l'axe longitudinal de chacune des deux portions du sangle à l'intérieur de l'évidement; et,
   - troisièmement soit parallèle à la surface de chacune des première et seconde portions de sangle à l'intérieur de l'évidement,
et de telle manière, en outre, que chacune des première et seconde portions de sangle soit bloquée en mouvement par rapport à la cale de stabilisation par pincement de ladite portion de sangle entre le pion et des portions respectives de la paroi interne de l'évidement se faisant face l'une à l'autre.

Dans un mode de réalisation directement complémentaire, les première et seconde portions de sangle peuvent passer dans le logement prévu dans la cale de stabilisation en sens opposés, de manière à se croiser dans ledit logement, et de manière pour la sangle à former une boucle dans un plan perpendiculaire à l'axe principal de la cale, avec deux ganses respectivement localisées de part et d'autre de la cale de stabilisation dans ledit plan et adaptées pour venir en engagement chacune avec l'une respective des apophyses épineuses de deux vertèbres à stabiliser.

Dans un mode de réalisation, l'évidement prévu dans la cale de stabilisation est de forme conique, et le pion de blocage est de forme conique complémentaire de la forme de l'évidement.

Dans un mode de réalisation, le corps de la cale de stabilisation comprend des passages pour la sangle s'étendant perpendiculairement à l'axe principal du corps de ladite cale, dont au moins un passage traversant l'évidement, dans lesquels passages au moins la sangle peut coulisser lorsqu'elle n'est pas bloquée en mouvement par rapport à la cale de stabilisation.

Dans un mode de réalisation, la sangle est une tresse réalisée en matériau textile à usage médical non résorbable.

Dans un mode de réalisation, l'évidement présente une zone d'ouverture taraudée de diamètre plus grand que le diamètre de l'évidement en arrière de ladite zone d'ouverture, et l'organe de verrouillage est une vis de même diamètre que le diamètre de la zone d'ouverture de l'évidement avec un filet adapté pour coopérer avec le taraudage de la zone d'ouverture, et avec une zone d'appui adaptée pour appuyer contre une zone de contact du pion de blocage dans l'évidement lorsque la vis est vissée dans la zone d'ouverture taraudée de l'évidement.

Dans un second aspect, l'invention concerne également un kit chirurgical comprenant un implant selon le premier aspect ci-dessus, et un outil d'aide au positionnement de la cale de l'implant, qui est aussi appelé porte-implant dans la suite, ainsi qu'une tige d'insertion du pion de blocage de l'implant.

Plus particulièrement, le kit est tel que :
- le porte-implant a un corps tubulaire présentant un canal interne avec un diamètre interne légèrement plus grand que le plus grand diamètre du pion de blocage de l'implant et adapté pour être fixé au corps de la cale de stabilisation de telle manière que le canal est en parallaxe avec l'axe de l'évidement dans le corps, et
- la tige d'insertion du pion de blocage adaptée pour coulisser dans le canal interne pour la mise en place du pion de blocage dans l'évidement du corps de la cale de stabilisation.

Dans un mode de réalisation, le corps tubulaire du porte-implant peut être muni à une première extrémité d'un filet pour coopérer avec le taraudage de l'évidement prévu dans le corps de la cale de stabilisation. Ceci permet la fixation du porte-implant à l'implant en utilisant le taraudage prévu par ailleurs pour la vis de verrouillage de l'implant qui n'est mise en place qu'ultérieurement pendant l'opération d'implantation.

Dans un autre mode de réalisation, le porte implant peut être muni à une seconde extrémité d'un taraudage à l'entrée du canal, et la tige d'insertion peut être munie d'un filet pour coopérer avec ledit taraudage de manière que l'insertion puis le vissage de la tige d'insertion dans le canal du porte implant entraîne le pion conique dans le logement prévu dans le corps de la cale.

### Brève Description des Dessins

D'autres caractéristiques et avantages de l'invention apparaîtront encore à la lecture de la description qui va suivre. Celle-ci est purement illustrative et doit être lue en regard des dessins annexés sur lesquels :
- la Figure 1, est une vue éclatée en trois dimensions d'une partie de l'implant intervertébral selon des modes de réalisation;
- la Figure 2 est une vue de face, suivant l'axe de la voie d'abord postérieure, de l'implant assemblé selon des modes de réalisation ;
- la Figure 3 est une vue éclatée en trois dimensions d'une partie de l'implant intervertébral et d'un porte-implant associé, selon des modes de réalisation ;
- la Figure 4 une vue éclatée en trois dimensions d'une partie de l'implant intervertébral avec le porte porte-implant monté sur l'implant, et d'une tige d'insertion du pion de blocage selon des modes de réalisation ;
- la Figure 5A et la Figure 5B sont une vue en coupe, et une vue en coupe en trois dimensions, respectivement, de la cale de stabilisation munie de la sangle et du pion de blocage installé, selon des modes de réalisation ;
- la Figure 6A et la Figure 6B sont des vues identiques à celles des Figures 5A et 5B, respectivement, montrant en outre la vis de verrouillage engagée à l'entrée de l'évidement dans la cale ; et,
- la Figure 7A et la Figure 7B sont des vues identiques à celles des Figures 5A et 5B, respectivement, montrant en outre la vis de verrouillage en position de verrouillage.

### Description détaillée de modes de réalisation

L'implant intervertébral est destiné à prendre place entre les apophyses épineuses de deux vertèbres adjacentes, c'est-à-dire des vertèbres consécutives dans l'empilement des vertèbres lombaires, dorsales et cervicales.

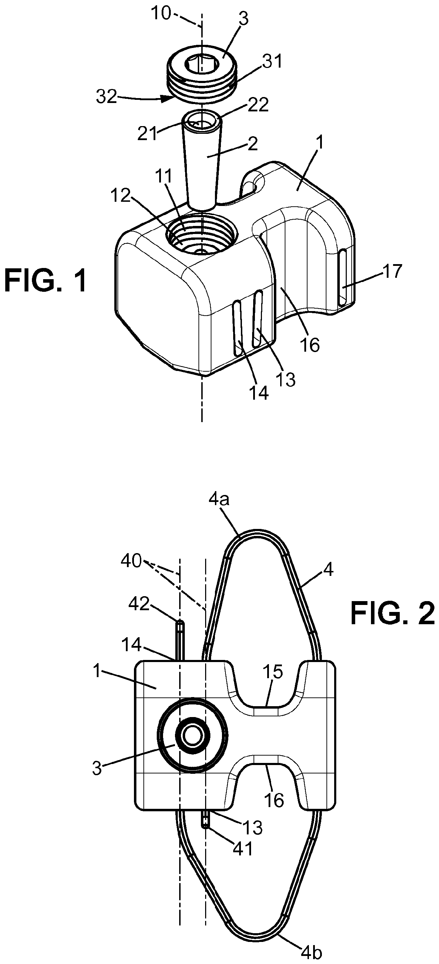

Les principaux éléments de l'implant intervertébral de stabilisation dynamique selon des modes de réalisation de la présente invention vont être décrits, tout d'abord, en référence aux Figures 1 et 2. Ainsi qu'il est montré sur ces figures, l'implant est composé d'une cale de stabilisation 1, d'un pion de blocage 2, d'une vis de verrouillage 3, et d'un lien souple 4.

La cale de stabilisation 1 comprend un corps globalement parallélépipédique avec un axe principal qui, pour des raisons de clarté de la Figure 1, est confondu sur cette figure axe l'axe longitudinal 10 d'un évidement 12 prévu dans le corps et sur lequel on reviendra plus loin. La Figure 2 est une vue de l'implant suivant l'axe longitudinal 10, lorsque l'implant est posé à plat contre les vertèbres du patient (lequel est allongé sur la table d'opération, sur le ventre). L'axe principal 10 coïncide alors avec l'axe de la voie d'abord postérieure, c'est-à-dire qu'il est perpendiculaire au dos du patient et donc à l'axe de la colonne vertébrale correspondant à la direction d'empilement des vertèbres depuis les vertèbres lombaires jusqu'aux vertèbres cervicales.

Pour ce qui est du vocabulaire descriptif, on considère dans la suite la direction d'observation du site d'implantation par le chirurgien suivant l'axe de la voie d'abord postérieure, lors de l'opération d'implantation et alors que le patient est allongé sur le ventre contre la table d'opération. Ainsi, la Figure 2 représente une vue de face suivant cet axe, et une vue de dessus suivant cette direction. Les termes « postérieurement », « avant » et « arrière », « devant » et « derrière », « avant » et « arrière », « dessus » et « dessous », « supérieur(e) » et « inférieur(e) », « haut » et « bas », « latéral(e) » et « côté », « droit(e) » et « gauche », notamment, sont utilisés dans la suite en référence à cette convention. Ces termes correspondent aussi au vocabulaire utilisé par les personnes de métier dans le domaine de la chirurgie du rachis.

Le corps de la cale de stabilisation 1 comprend, d'un côté latéral du parallélépipède, plus particulièrement à droite sur les Figures 1 et 2, une échancrure ou encoche supérieure 15 et une échancrure ou encoche inférieure 16. Ces échancrures sont adaptées pour venir en appui contre deux vertèbres à stabiliser, et plus particulièrement sur l'apophyse de la vertèbre du dessus par l'encoche 15 et sur l'apophyse de la vertèbre du dessous par l'encoche 16, respectivement. Dit autrement, dans la position installée de l'implant pour assurer la stabilisation de deux vertèbres adjacentes, les apophyses épineuses de ces vertèbres se logent dans les échancrures 15 et 16 du corps de la cale 1.

Ainsi qu'il est visible sur la Figure 1, le parallélépipède du corps de la cale 1 présente des angles adoucis (i.e. arrondis), au moins sur la face arrière destinée à être recouverte par les chairs et la peau du dos du patient. Ceci limite les risques d'inflammation ou d'endommagement des chairs du dos en contact avec l'implant. Egalement, cela réduit la gêne voire la douleur que peut ressentir le patient en cas d'appui sur cette partie de son dos, par exemple lorsqu'il est adossé contre un support (par exemple un dossier de siège) ou est allongé sur le dos (par exemple sur une surface dure comme le sol).

Le pion de blocage 2 peut avoir une forme cylindrique et conique, c'est-à-dire avoir la forme d'un cylindre dont le diamètre de la section (circulaire) se réduit progressivement suivant son axe longitudinal. Sur la Figure 1, toujours pour des raisons de clarté du dessin, l'axe longitudinal du pion coïncide avec l'axe principal 10 du corps 1 de la cale et avec l'axe longitudinal de l'évidement 12.

Le pion 2 est adapté pour coopérer avec l'évidement 12 prévu dans le corps de la cale 1, par exemple du côté latéral du parallélépipède opposé à celui où se trouvent les échancrures 15 et 16 (i.e., à gauche sur les Figures 1 et 2). A cet effet, le pion 2 et l'évidement 12 ont des formes complémentaires l'une de l'autre. Dans l'exemple représenté, le pion a la forme d'un cylindre conique, et l'évidement 12 a également une forme de cylindre conique en creux avec un angle d'ouverture égal à celui du pion et un diamètre d'ouverture légèrement plus grand que celui du pion.

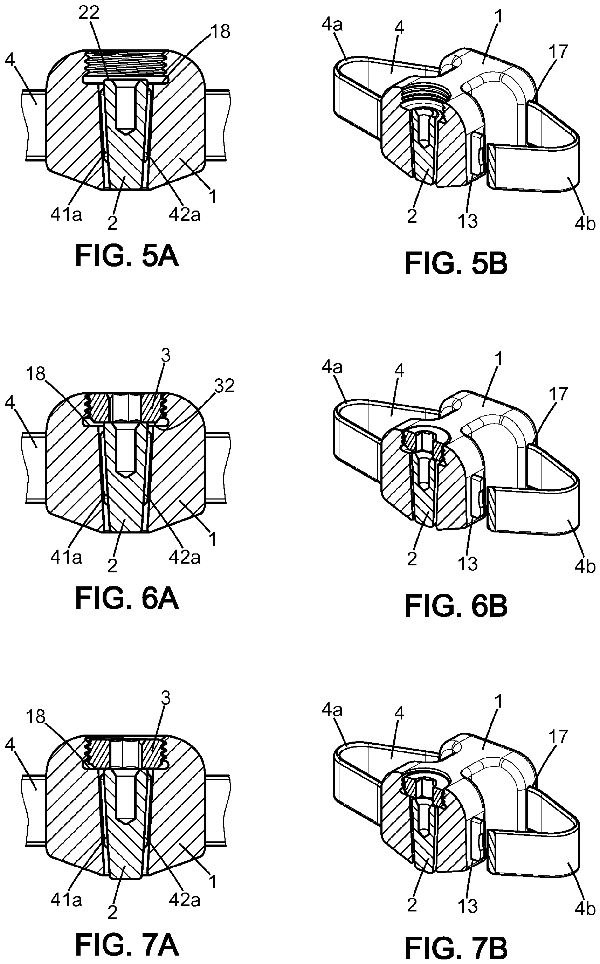

L'évidement est débouchant (ouvert) au moins du côté de la face arrière de la cale 1, et de préférence du côté de chacune des faces avant et arrière de la cale, ainsi qu'il est montré sur les Figures 5A-5B, 6A-6B, et 7A-7B. Le diamètre de l'ouverture est sensiblement plus grand que le plus grand diamètre du pion, et est constant dans une zone d'entrée de l'évidement du côté de la face arrière, par laquelle le pion 2 est destiné à entrer par son extrémité effilée. Dans cette zone d'entrée de l'évidement, ses parois présentent un filetage intérieur (taraudage) 11 pour la vis de verrouillage sur lequel on reviendra plus loin. La longueur de la zone d'entrée, suivant l'axe longitudinale, est au moins égale à l'épaisseur de la vis de verrouillage 3. En avant de cette zone d'entrée, la forme de l'évidement est conique, sans taraudage (parois lisses) et correspond sensiblement à la forme complémentaire du pion, c'est-à-dire que la forme est conique avec le même angle d'ouverture que celui du pion mais avec un diamètre légèrement plus grand pour pouvoir recevoir le pion et la tresse comme il sera explicité plus loin. Dit autrement, en avant de la zone d'entrée de l'évidement qui est taraudée, le diamètre de l'évidement décroit progressivement suivant son axe longitudinal 10 vers l'avant. Notamment, la longueur de la partie conique de l'évidement suivant l'axe longitudinal de l'évidement est sensiblement égale à la longueur du pion de blocage 2 suivant sa direction longitudinale.

La vis de verrouillage 3 est également représentée à la Figure 1 en parallaxe avec l'axe principal 10 du corps 1 de la cale et l'axe longitudinal de l'évidement 12. La vis 3 a un diamètre plus grand que le plus grand diamètre du pion. Ce diamètre correspond à celui de la zone d'ouverture taraudée de l'évidement 12. La vis 3 présente un filetage extérieur (filet) 31, adapté pour coopérer avec le taraudage 11 de l'évidement 12.

La vis 3 présente en outre une zone d'appui 32, tournée vers le bas sur la Figure 1, pouvant venir en appui contre une zone de contact 22 à l'arrière du pion 2. Dans un mode de réalisation la zone d'appui 32 de la vis 3 et/ou la zone de contact 22 de pion 2 sont des surfaces planes circulaires.

La fonction de la vis 3 est de verrouiller la position du pion 2 engagé dans l'évidement 12. Une autre fonction de la vis est, selon des modes de réalisation, de régler la position axiale du pion dans l'évidement : en tournant la vis 3 engagée dans l'évidement 12 via leurs filetages respectifs, la vis appuie par sa surface 32 contre une surface de contact 22 du côté arrière du pion 2, en sorte que celui progresse dans l'évidement 12 suivant son axe longitudinal, depuis la face arrière de la cale vers sa face avant. Des détails sur ce réglage en position seront donnés plus loin en référence aux Figures 5A-5B, 6A-6B, et 7A-7B.

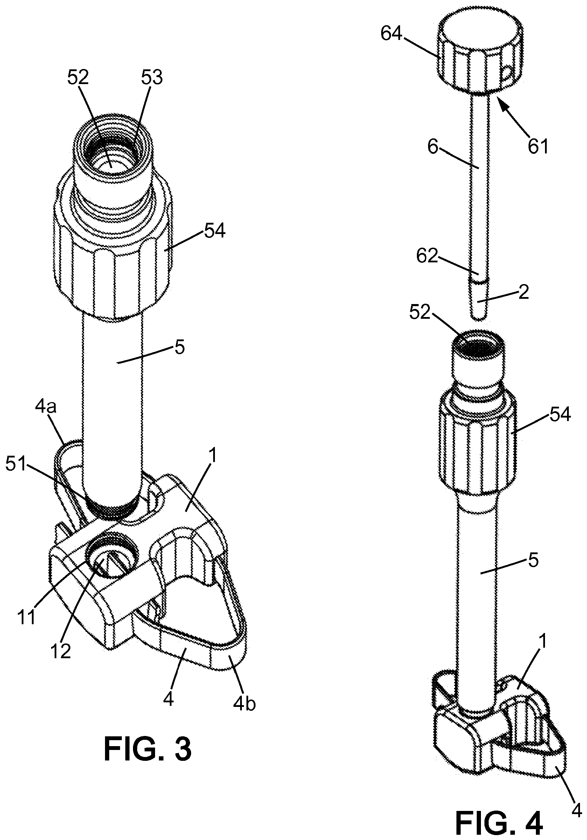

Le lien souple 4 peut être une tresse réalisée dans un matériau textile à usage médical (non résorbable), par exemple en Polyéthylène téréphtalate (PET) ou en Polyéthylène (PE). Ces matériaux peuvent être choisis en raison de leur biocompatibilité et de leur grande inertie chimique. En position installée de l'implant, le lien souple 4 enserre les apophyses d'une manière similaire à celle décrite dans le document EP 2192863, en particulier comme le montrent les Figures 3, 10 et 11A de la publication de ce document.

Le lien souple 4 a de préférence la forme d'une sangle (i.e., d'un ruban), avec un axe longitudinal et une surface de sangle s'étendant suivant ladite direction longitudinale. Il peut passer dans un passage 17 du corps de la cale prévu dans l'extrémité caudale du corps de la cale du côté droit de celle-ci, c'est-à-dire du côté des échancrures 15 et 16 qui est opposé à l'évidement 12. Le passage 17 traverse le corps de la cale 1 perpendiculairement à l'axe principal 10 du corps 1 de la cale. Lorsqu'il n'est pas bloqué en mouvement par rapport à la cale, le lien souple 4 peut coulisser dans le passage 17.

Le lien souple 4 peut également passer dans l'évidement 12. A cet effet, le corps de la cale peut présenter deux autres passages 13 et 14 traversant le corps 1 de part en part perpendiculairement à l'axe principal 10 du corps 1 de la cale. Les passages 13 et 14 s'étendent donc perpendiculairement à l'axe principal 10 du corps 1 de la cale. L'un au moins des passages 13 et 14, et de préférence les deux passages 13 et 14, passent par l'évidement 12. Dit autrement, les gorges 13 et 14 débouchent dans l'évidement, chacune par le côté avant et par le côté arrière. Dans le mode de réalisation représenté, les deux passages 13 et 14 passent ainsi par l'évidement 12, mais cela n'est pas obligatoire, et il serait possible qu'un seul des passages 13 et 14 seulement traverse l'évidement 12. Lorsqu'il n'est pas bloqué en mouvement par rapport à la cale 1, le lien souple 4 peut coulisser dans les passages 13 et 14.

Le lien souple 4 est inséré manuellement par le chirurgien, par exemple d'abord au travers du passage 17. Puis les deux extrémités 41 et 42 du lien souple 4 sont tour à tour insérées dans les gorges 13 et 14 après les avoir engagées par-dessus et par-dessous les apophyses épineuses des vertèbres supérieure et inférieure, respectivement.

Plus particulièrement, la sangle forme alors une boucle dans un plan perpendiculaire à l'axe principal 10 de la cale 1, avec au moins une et de préférence deux ganses 4a et 4b respectivement localisées de part et d'autre de la cale dans ledit plan. Ces ganses 4a et 4b de la tresse textile sont adaptées pour venir en engagement chacune avec l'une respective des apophyses épineuses des deux vertèbres à stabiliser.

Le pion de blocage 2 peut venir en engagement avec la cale de stabilisation suivant la direction de l'axe principal 10 du corps 1 de la cale entre chacune des portions de sangle à l'intérieur de l'évidement, de telle manière que l'axe longitudinal du pion :
- premièrement coïncide alors avec l'axe longitudinal 10 de l'évidement;
- deuxièmement soit perpendiculaire à l'axe longitudinal 40 de chacune des deux portions du sangle à l'intérieur de l'évidement; et,
- troisièmement soit parallèle à la surface de chacune des deux portions de sangle à l'intérieur de l'évidement (i.e., des portions de la sangle du côté des extrémités respectivement 41 et 42, de la sangle).

En outre, chacune des portions de la sangle du côté de ses extrémités respectivement 41 et 42, est alors bloquée en mouvement par rapport à la cale 1. Ce blocage est obtenu par pincement desdites portions de sangle entre le pion et des portions respectives de la paroi interne de l'évidement 12 se faisant face l'une à l'autre.

Pour illustrer ce blocage des deux portions de la sangle 4 par le pion 2, la partie centrale des Figures 5A, 6A et 7A correspondant à l'espace interne de l'évidement (qui reçoit le pion 2) est représentée suivant un plan de coupe à 90 degrés par rapport au plan de coupe du corps 1 de la cale sur ces même figures. Ainsi, ces Figures 5A, 6A et 7A montrent en leur centre une vue en coupe des portions 41a et 42a de la sangle 40 du côté des extrémités 41 et 42 de ladite sangle, respectivement.

Dans un mode de réalisation, ces deux portions extrémales de la sangle 41a et 42a peuvent passer dans le logement 12 prévu dans la cale 1 en sens opposés, de manière à se croiser dans ledit logement et à former les ganses 4a et 4b. Ce mode de réalisation est montré aux Figures. Il n'est cependant pas exclusif. En effet, les deux portions extrémales 41a et 41b de la sangle pourraient passer parallèlement l'une à l'autre dans les passages 13 et 14, et donc dans l'évidement 12, moyennant une autre forme d'engagement avec les apophyses épineuses des vertèbres à stabiliser, et moyennant également une autre forme de la cale de stabilisation. En particulier, dans un tel mode de réalisation, la sangle ne formerait qu'une ganse, en sorte que deux sangles seraient utilisées, à savoir une pour chacune des vertèbres, avec la même cale.

Pour faciliter l'opération d'insertion dans les passages 13 et 14, les extrémités 41 et 42 de la sangle peuvent être renforcées, par exemple par traitement avec une soudeuse à ultrasons, par ajout de matière, ou par un embout en titane, ou en tout autre matériau approprié, ou par tout autre moyen équivalent.

Avantageusement, les moyens de blocage et de verrouillage du lien souple 4, qui comprennent le pion 2 et la vis 3, sont mis en place par la face postérieure de la cale de stabilisation 1. Cette disposition permet au chirurgien de réduire la taille de l'incision au strict minimum, et ainsi de conserver l'intégrité des tissus organiques environnants, notamment les muscles du dos.

Après la mise en place du lien souple 4 et sa mise en tension à l'aide d'un outil approprié (opération non décrite dans le cadre de la présente description), le pion de blocage 2 est inséré dans le logement 12, entre les deux portions extrémales du lien souple 4. L'insertion du pion 2 provoque le serrage des portions du lien souple 4 entre la surface circonférentielle externe du pion 2 et la paroi interne en regard de l'évidement 12, et stabilise le lien souple 4. En raison de cette compression du lien souple 4, celui-ci ne peut plus coulisser dans les passages 13, 14 et 17. La vis de verrouillage 3 est alors engagée dans l'évidement 12, par exemple à la main (à l'aide ou non d'un porte-outil), une fois que le pion 2 a été entièrement inséré dans la portion taraudée 11 de l'évidement 12 comme montré aux Figures 5A et 5B. Cette opération est illustrée par les Figures 6A et 6B. La fonction de la vis 3 est de verrouiller (en position axiale) l'engagement du pion de blocage 2 dans l'évidement 12.

La méthode de mise en place du pion de blocage 2 fait appel à un porte implant 5 et à une tige d'insertion 6, qui sont montrés aux Figures 3 et 4. Le porte implant 5 est par exemple un tube creux, c'est-à-dire qu'il a un corps tubulaire présentant un canal interne 52, par exemple de section circulaire constante avec un diamètre interne légèrement plus grand que le plus grand diamètre du pion de blocage 2.

A l'une des extrémités du tube 5, il peut être prévu un taraudage 53 à l'entrée du canal 52. Egalement, des moyens de préhension 54 pouvant avoir la forme d'une collerette cannelée entourant le tube 5 comme représenté, peuvent être prévus du côté de cette extrémité d'entrée du tube 5.

A l'autre de ses extrémités, le tube 5 est muni d'un filetage extérieur 51 qui coopère avec le taraudage 11 de la cale de stabilisation 1. Le porte implant 5 peut ainsi être vissé dans la cale de stabilisation 1, avec le canal interne 52 dans aligné avec l'axe longitudinal 10 de l'évidement 2, c'est-à-dire en parallaxe avec l'évidement 12, en vue de l'insertion du pion de blocage 2 dans ledit évidement 12.

A cet effet, le pion 2 peut être fixé de manière facilement détachable à l'extrémité de la tige d'insertion 6 au moyen par exemple d'un adaptateur élastique, non représenté, et dont la description détaillée n'est pas nécessaire à la bonne compréhension du principe de fonctionnement.

La tige d'insertion 6 avec le pion conique 2 ainsi maintenu de manière détachable à son extrémité, sont introduits dans le canal 52 du porte implant 5. La tige d'insertion 6 est munie d'un filet externe en dessous de moyens de préhension tels qu'une tête cannelée 61 comme représenté. Ce filet (non représenté) coopère avec le taraudage 53 du porte implant 5. De cette manière, l'insertion puis le vissage de la tige d'insertion 6 dans le canal 52 du porte implant 5 entraîne le pion conique 2 dans le logement 12 de la cale 1. On peut ainsi engager le pion 2 dans la portion conique et non taraudée de l'évidement 12, ainsi qu'il a été indiqué plus haut, par vissage de la tige d'insertion dans le porte-implant. Après vissage complet de la tige d'insertion dans le porte-implant, le pion 2 est complètement engagé dans la portion conique de l'évidement 12.Il vient alors en engagement avec la paroi interne de l'évidement en pinçant le lien souple 4 contre cette paroi. L'écrasement de la tresse qui résulte de ce pincement donne une certaine élasticité, très faible, à l'engagement du pion contre la paroi interne de l'évidement.

Le porte implant 5 peut alors être dévissé de la cale 1, éventuellement après dévissage de la tige d'insertion du porte-implant (non obligatoire). Le pion 2 se détache alors automatiquement de l'extrémité de la tige d'insertion 6 en restant en place dans la portion conique non taraudée de l'évidement 12. A cet effet, la force de la liaison élastique du pion 2 à l'extrémité 62 de la tige d'insertion 2 est calibrée de manière à être inférieure à la pression de serrage appliquée sur le pion 2 par la paroi interne de la portion conique non taraudée de l'évidement 12 lorsque le pion y est totalement engagé.

Lors de l'opération d'insertion du pion 2 dans l'évidement 12, la cale de stabilisation 1 peut être maintenue par le chirurgien, en position avec les ganses 4a et 4b de la sangle 4 engagées autour des apophyses épineuses des vertèbres à stabiliser, grâce notamment à la zone de préhension du porte-implant 5. Le porte-implant sert donc simultanément au maintien de la cale de stabilisation en position contre les vertèbres lors de l'étape critique consistant à insérer le pion de blocage du lien souple 4, et de guide pour l'insertion du pion grâce à la tige 6 qui coulisse dans le canal interne 52 du porte-implant et se visse sur celui-ci.

Une fois le porte-implant 5 désolidarisé de la cale 1 qui est déjà ainsi en situation de stabilisation des vertèbres, la vis de verrouillage 3 peut être engagée dans la portion taraudée 11 de l'évidement 12 prévu dans la cale, comme montré à la Figure 6A et à la Figure 6B. On notera que, à cette étape, le lien souple est déjà bloqué par le pion 2, par pincement des portions extrémales 41a et 42a contre des portions opposées de la paroi interne de l'évidement 12, comme montré à la Figure 5A et à la Figure 5B.

La vis 3 et le pion conique 2 permettent de maîtriser la pression de serrage exercée sur le lien souple 4 sans avoir recours à un dispositif de mesure de la force ou du couple de serrage.

A cet effet, la longueur du pion conique 2 suivant sa direction longitudinale est calibrée pour que sa surface plane de contact 22 affleure très légèrement au-dessus du lamage du trou taraudé 11 de la cale de stabilisation 1 lorsqu'il est inséré selon la technique décrite précédemment. La face d'appui plane 32 de la vis 3 vient alors en appui sur le lamage du trou taraudé 11, ainsi qu'en appui sur la surface plane de contact 22 du pion conique 2, du côté arrière, comme montré à la Figure 7A et à la Figure 7B.

Le vissage de la vis 3 dans le trou taraudé 11 s'effectue par exemple à la main, à l'aide d'un tournevis, par exemple à tête hexagonale. Lorsque la surface plane 32 de la vis de serrage entre en contact avec la surface de contact 22 du verrou de blocage, la vis de serrage entraîne le pion de blocage 2 plus en avant dans l'évidement 12, jusqu'à ce que la surface plane 32 de la vis de serrage rencontre le lamage du trou taraudé 11 qui forme un épaulement. La progression axiale du pion conique 2 est alors bloquée par cet épaulement, assurant que la pression exercée par le pion conique 2 sur le lien souple 4 n'augmentera plus même si le chirurgien continue d'exercer un couple de serrage sur la vis de serrage 3 à l'aide du tournevis.

Dans cette position, la vis 3 empêche tout mouvement du pion 2 qui tendrait à le faire ressortir de l'évidement 12, par exemple sous l'effet de tensions exercées sur le lien souple 4 par les mouvements du patient dans les gestes de la vie courante. C'est la fonction de verrouillage de la vis 3 conformément au principe des modes de réalisation de l'invention. Ainsi qu'on l'a compris, cette fonction s'exerce de manière radicalement différente de celle de la vis décrite dans les documents EP 2192863 et EP 2303163. Elle s'exerce en coopération avec le pion conique 2 qui est fonctionnellement et structurellement radicalement différent de la pièce mobile de l'implant décrit dans ces documents. L'originalité de la fonction du pion de blocage selon des modes de réalisation de l'invention réside dans la direction de déplacement du pion qui est orthogonale à la direction de la force d'appui du pion contre la surface de la sangle 4 et contre la paroi interne de l'évidement 12 prévu dans la cale.

La cale de stabilisation 1 peut être réalisée en polymère, par exemple en polyéther éther cétone (PEEK). Elle peut être obtenue par usinage à partir d'une barre ou d'un bloc de matériau brut, par injection moulée, par impression 3D, ou par toute autre technique équivalente.

Le pion de blocage conique 2 est de préférence réalisé en alliage de titane, choisi pour sa résistance mécanique et sa biocompatibilité. Il est muni d'une extrémité arrondie du côté le plus effilé pour faciliter son passage entre les portions de sangle en tension du lien souple. Il peut par exemple être obtenu par usinage à partir d'une barre de matériau brut.

La vis 3 de serrage et de verrouillage peut également être en alliage de titane, pour les mêmes raisons et avec les mêmes avantages. Elle peut être obtenue par usinage à partir d'une barre de matériau brut.

Le lien souple 4 est fabriqué, de préférence, en textile tressé. Ainsi qu'il a déjà été dit plus haut, les extrémités du lien souple peuvent être rigidifiées de manière à faciliter leur préhension et leur guidage au travers des passages 13, 14 et 17 prévus dans le corps de la cale de stabilisation 1.

La présente invention se distingue de l'invention décrite dans le document EP 2192863 en particulier en raison de ce que la cale de stabilisation dynamique ne comporte pas de partie mise en mouvement perpendiculairement à la surface de la sangle, donc suivant une direction latérale. Au contraire, le pion se déplace uniquement suivant l'axe de la voie d'abord postérieure. La solution décrite dans la présente description permet en outre d'installer une vis de verrouillage 3 qui procure une sécurité (verrouillage en position) du pion de blocage 2 de la sangle 4, et permet un contrôle de la pression exercée en raison de la maîtrise du déplacement axial du pion 2 dans l'évidement 12. Cette vis est également mise en place et serrée suivant la direction de l'axe de la voie d'abord postérieure.

En d'autres termes, et suivant le principal avantage des modes de réalisations décrits, aucune action du chirurgien ni aucun mouvement de pièce mobile n'est effectué suivant une direction latérale. Tout se passe suivant l'axe de la voie d'abord postérieure.

L'invention a été décrite et illustrée dans la présente description détaillée et dans les Figures, dans des formes de réalisation particulièrement avantageuses. Elle ne se limite pas, toutefois aux formes de réalisation présentées. D'autres variantes et modes de réalisation peuvent être déduits et mis en oeuvre par la personne du métier à la lecture de la présente description et des dessins annexés.

Dans les revendications, les termes « comprend » ou « comporte » n'excluent pas d'autres éléments ou d'autres étapes. Les différentes caractéristiques présentées et/ou revendiquées peuvent être avantageusement combinées. Leur présence dans la description ou dans des revendications dépendantes différentes, n'excluent pas cette possibilité. Les signes de référence ne sauraient être compris comme limitant la portée de l'invention.

## Revendications

1. Implant intervertébral comprenant :
- une cale de stabilisation (1) adaptée pour stabiliser entre elles au moins deux vertèbres adjacentes par interposition entre des apophyses épineuses desdites vertèbres, ayant un corps sensiblement parallélépipédique qui présente un axe principal déterminé,
- au moins une sangle (4) formant lien souple pour la fixation de la cale de stabilisation aux apophyses épineuses des vertèbres à stabiliser, ladite sangle comportant une première et une seconde portions de sangle (41a,42a) comprenant chacune l'une des extrémités opposées (41,42) de la sangle, et ladite sangle présentant une direction longitudinale déterminée et une surface de sangle déterminée,
- au moins un ensemble mobile (2) adapté pour venir en engagement avec la cale de stabilisation, de manière à bloquer la sangle en mouvement par rapport à la cale de stabilisation par pincement de ladite sangle entre ledit ensemble mobile et ladite cale de stabilisation, et
- un organe de verrouillage (3) pour verrouiller en position axiale l'engagement de l'ensemble mobile avec la cale de stabilisation et ainsi le blocage de la sangle, ***caractérisé en ce que***
- le corps de la cale de stabilisation comprend au moins un évidement (12) dans lequel peut passer au moins la première portion de sangle, ledit au moins un évidement ayant un axe longitudinal parallèle à l'axe principal de la cale de stabilisation, et une paroi interne s'étendant parallèlement audit axe longitudinal de l'évidement avec une forme déterminée;
- l'ensemble mobile (2) comprend au moins un pion de blocage ayant un axe longitudinal déterminé et une forme déterminée sensiblement complémentaire de la forme de l'évidement, pour venir en engagement avec la cale de stabilisation par déplacement suivant la direction de l'axe principal du corps de ladite cale à l'intérieur de l'évidement de telle manière que l'axe longitudinal du pion de blocage :
- premièrement coïncide alors avec l'axe longitudinal de l'évidement ;
- deuxièmement est perpendiculaire à l'axe longitudinal de la première portion de sangle à l'intérieur de l'évidement ; et,
- troisièmement est parallèle à la surface de la première portion de sangle à l'intérieur de l'évidement,
et de telle manière, en outre, que la portion de sangle à l'intérieur de l'évidement est bloquée en mouvement par rapport à la cale de stabilisation par pincement de ladite portion de sangle entre le pion de blocage et la paroi interne du corps de la cale de stabilisation délimitant ledit évidement.

2. Implant intervertébral selon la revendication 1, dans lequel :
- chacune des première et seconde portions de sangle peut passer dans le logement prévu dans la cale de stabilisation, de manière pour la sangle à former au moins une boucle dans un plan perpendiculaire à l'axe principal de la cale, avec une ganse adaptée pour venir en engagement avec l'une des apophyses épineuses de deux vertèbres à stabiliser, et dans lequel, en outre
- le pion de blocage peut venir en engagement avec la cale de stabilisation suivant la direction de l'axe principal du corps de ladite cale entre chacune des première et seconde portions de sangle (41a,42a) à l'intérieur de l'évidement, de telle manière que l'axe longitudinal du pion :
- premièrement coïncide alors avec l'axe longitudinal de l'évidement ;
- deuxièmement soit perpendiculaire à l'axe longitudinal de chacune des deux portions du sangle à l'intérieur de l'évidement; et,
- troisièmement soit parallèle à la surface de chacune des première et seconde portions de sangle à l'intérieur de l'évidement,
et de telle manière, en outre, que chacune des première et seconde portions de sangle soit bloquée en mouvement par rapport à la cale de stabilisation par pincement de ladite portion de sangle entre le pion et des portions respectives de la paroi interne du corps de la cale de stabilisation délimitant ledit évidement se faisant face l'une à l'autre.

3. Implant intervertébral selon la revendication 2, dans lequel les première et seconde portions de sangle peuvent passer dans le logement prévu dans la cale de stabilisation en sens opposés, de manière à se croiser dans ledit logement, et de manière pour la sangle à former une boucle dans un plan perpendiculaire à l'axe principal de la cale, avec deux ganses respectivement localisées de part et d'autre de la cale de stabilisation dans ledit plan et adaptées pour venir en engagement chacune avec l'une respective des apophyses épineuses de deux vertèbres à stabiliser.

4. Implant intervertébral selon l'une quelconque des revendications 1 à 3, dans lequel l'évidement prévu dans la cale de stabilisation est de forme conique, et dans lequel le pion de blocage est de forme conique complémentaire de la forme de l'évidement.

5. Implant intervertébral selon l'une quelconque des revendications 1 à 4, dans lequel le corps de la cale de stabilisation comprend des passages (13,14) pour la sangle (4) s'étendant perpendiculairement à l'axe principal (10) du corps (1) de ladite cale, dont au moins un passage traversant l'évidement (12), dans lesquels passages au moins la sangle peut coulisser lorsqu'elle n'est pas bloquée en mouvement par rapport à la cale de stabilisation.

6. Implant intervertébral selon l'une quelconque des revendications 1 à 5, dans lequel la sangle (4) est une tresse réalisée en matériau textile à usage médical non résorbable.

7. Implant intervertébral selon l'une quelconque des revendications 1 à 6, dans lequel l'évidement présente une zone d'ouverture taraudée (11) de diamètre plus grand que le diamètre de l'évidement en arrière de ladite zone d'ouverture, et dans lequel l'organe de verrouillage est une vis de même diamètre que le diamètre de la zone d'ouverture de l'évidement avec un filet adapté pour coopérer avec le taraudage de ladite zone d'ouverture, et avec une zone d'appui (32) adaptée pour appuyer contre une zone de contact (22) du pion de blocage (2) dans l'évidement (12) lorsque la vis est vissée dans la zone d'ouverture taraudée de l'évidement.

8. Kit chirurgical comprenant :
- un implant intervertébral selon l'une quelconque des revendications précédentes,
- un porte-implant (5) avec un corps tubulaire présentant un canal interne (52) avec un diamètre interne légèrement plus grand que le plus grand diamètre du pion de blocage (2) de l'implant et adapté pour être fixé au corps de la cale de stabilisation de telle manière que le canal est en parallaxe avec l'axe de l'évidement (12) dans ledit corps, et
- une tige (6) d'insertion du pion de blocage adaptée pour coulisser dans le canal interne pour la mise en place du pion de blocage dans l'évidement du corps de la cale de stabilisation.

9. Kit chirurgical selon la revendication 8 limité en ce qu'il correspond à un implant intervertébral selon la revendication 7, dans lequel le corps tubulaire du porte-implant (5) est muni à une première extrémité d'un filet (51) pour coopérer avec le taraudage (11) de l'évidement (12) prévu dans le corps de la cale de stabilisation (1).

10. Kit chirurgical selon l'une des revendications 8 ou 9, dans lequel le porte implant est muni à une seconde extrémité d'un taraudage (53) à l'entrée du canal (52), et dans lequel la tige d'insertion (6) est munie d'un filet (61) pour coopérer avec ledit taraudage de manière que l'insertion puis le vissage de la tige d'insertion (6) dans le canal (52) du porte implant (5) entraîne le pion conique (2) dans le logement (12) prévu dans le corps de la cale (1).

## Patentansprüche

1. Zwischenwirbelimplantat, umfassend:
- einen Stabilisierungskeil (1), der dazu geeignet ist, mindestens zwei benachbarte Wirbel durch Zwischenlage zwischen Dornfortsätzen dieser Wirbel miteinander zu stabilisieren, und der einen im Wesentlichen parallelepipedischen Körper aufweist, der eine bestimmte Hauptachse aufweist,
- mindestens einen Gurt (4), der eine flexible Verbindung zur Befestigung des Stabilisierungskeils an den Dornfortsätzen der zu stabilisierenden Wirbel bildet, wobei der Gurt einen ersten und einen zweiten Gurtabschnitt (41a, 42a) aufweist, die jeweils eines der gegenüberliegenden Enden (41, 42) des Gurts umfassen, und wobei der Gurt eine bestimmte Längsrichtung und eine bestimmte Gurtfläche aufweist,
- mindestens eine bewegliche Anordnung (2), die so beschaffen ist, dass sie mit dem Stabilisierungskeil in Eingriff kommt, um den Gurt bei der Bewegung in Bezug auf den Stabilisierungskeil durch Einklemmen des Gurts zwischen der beweglichen Anordnung und dem Stabilisierungskeil zu blockieren, und
- eine Verriegelungsvorrichtung (3), um den Eingriff der beweglichen Anordnung mit dem Stabilisierungskeil und damit die Blockierung des Gurts in axialer Position zu verriegeln,
**dadurch gekennzeichnet, dass**
- der Körper des Stabilisierungskeils mindestens eine Aussparung (12) umfasst, durch die mindestens der erste Gurtabschnitt verlaufen kann, wobei die mindestens eine Aussparung eine Längsachse parallel zur Hauptachse des Stabilisierungskeils und eine Innenwand aufweist, die sich parallel zu der Längsachse der Aussparung mit einer bestimmten Form erstreckt;
- die bewegliche Anordnung (2) mindestens einen Blockierstift mit einer bestimmten Längsachse und einer bestimmten Form umfasst, die im Wesentlichen komplementär zur Form der Aussparung ist, um mit dem Stabilisierungskeil in Eingriff zu kommen, indem der Körper des Keils innerhalb der Aussparung in Richtung der Hauptachse verschoben wird, so dass die Längsachse des Blockierstifts:
- erstens dann mit der Längsachse der Aussparung zusammenfällt;
- zweitens senkrecht zur Längsachse des ersten Gurtabschnitts innerhalb der Aussparung ist; und,
- drittens parallel zur Oberfläche des ersten Gurtabschnitts innerhalb der Aussparung ist,
und ferner derart, dass der Gurtabschnitt innerhalb der Aussparung gegen eine Bewegung relativ zum Stabilisierungskeil durch Einklemmen des Gurtabschnitts zwischen dem Blockierstift und der Innenwand des Körpers des Stabilisierungskeils, der die Aussparung begrenzt, blockiert wird.

2. Zwischenwirbelimplantat nach Anspruch 1, wobei:
- jeder der ersten und zweiten Gurtabschnitte durch die in dem Stabilisierungskeil vorgesehene Aufnahme verlaufen kann, so dass der Gurt in einer Ebene senkrecht zur Hauptachse des Keils mindestens eine Schleife mit einem Band bildet, das so beschaffen ist, dass es mit einem der Dornfortsätze von zwei zu stabilisierenden Wirbeln in Eingriff kommt, und wobei ferner
- der Verriegelungsstift mit dem Stabilisierungskeil entlang der Richtung der Hauptachse des Körpers des Keils zwischen jedem der ersten und zweiten Gurtabschnitte (41a, 42a) innerhalb der Aussparung in Eingriff kommen kann, so dass die Längsachse des Stifts:
- erstens dann mit der Längsachse der Aussparung zusammenfällt;
- zweitens senkrecht zur Längsachse jedes der beiden Gurtabschnitte innerhalb der Aussparung ist; und,
- drittens parallel zur Oberfläche jedes der ersten und zweiten Gurtabschnitte innerhalb der Aussparung ist,
und ferner derart, dass jeder der ersten und zweiten Gurtabschnitte gegen eine Bewegung relativ zum Stabilisierungskeil durch Einklemmen des Gurtabschnitts zwischen dem Stift und jeweiligen Abschnitten der Innenwand des Körpers des Stabilisierungskeils, die die Aussparung begrenzen, die einander zugewandt sind, blockiert wird.

3. Zwischenwirbelimplantat nach Anspruch 2, bei dem der erste und der zweite Gurtabschnitt in entgegengesetzter Richtung durch die im Stabilisierungskeil vorgesehene Aussparung verlaufen können, so dass sie sich in der Aussparung kreuzen und der Gurt in einer Ebene senkrecht zur Hauptachse des Keils eine Schleife bildet, mit zwei Bändern, die jeweils auf beiden Seiten des Stabilisierungskeils in der Ebene angeordnet und so beschaffen sind, dass sie jeweils mit einem der Dornfortsätze von zwei zu stabilisierenden Wirbeln in Eingriff kommen.

4. Zwischenwirbelimplantat nach einem der Ansprüche 1 bis 3, wobei die in dem Stabilisierungskeil vorgesehene Aussparung konisch geformt ist und wobei der Verriegelungsstift eine zur Form der Aussparung komplementäre konische Form aufweist.

5. Zwischenwirbelimplantat nach einem der Ansprüche 1 bis 4, wobei der Körper des Stabilisierungskeils Durchgänge (13, 14) für den Gurt (4) aufweist, die sich senkrecht zur Hauptachse (10) des Körpers (1) des Keils erstrecken, wobei mindestens ein Durchgang durch die Aussparung (12) verläuft, wobei in den Durchgängen mindestens der Gurt gleiten kann, wenn er nicht gegen eine Bewegung relativ zum Stabilisierungskeil blockiert ist.

6. Zwischenwirbelimplantat nach einem der Ansprüche 1 bis 5, wobei der Gurt (4) ein Geflecht ist, das aus einem nicht resorbierbaren textilen Material für medizinische Zwecke hergestellt ist.

7. Zwischenwirbelimplantat nach einem der Ansprüche 1 bis 6, wobei die Aussparung einen Öffnungsbereich mit Innengewinde (11) aufweist, dessen Durchmesser größer ist als der Durchmesser der Aussparung hinter dem Öffnungsbereich, und wobei die Verriegelungsvorrichtung eine Schraube mit demselben Durchmesser wie der Durchmesser des Öffnungsbereichs der Aussparung ist, mit einem Gewinde, das dazu geeignet ist, mit dem Innengewinde des Öffnungsbereichs zusammenzuwirken, und mit einem Anschlagbereich (32), der dazu geeignet ist, gegen einen Kontaktbereich (22) des Blockierstifts (2) in der Aussparung (12) zu drücken, wenn die Schraube in den mit Innengewinde versehenen Öffnungsbereich der Aussparung eingeschraubt wird.

8. Chirurgisches Kit, umfassend:
- ein Zwischenwirbelimplantat nach einem der vorhergehenden Ansprüche,
- einen Implantathalter (5) mit einem röhrenförmigen Körper, der einen inneren Kanal (52) mit einem Innendurchmesser aufweist, der etwas größer ist als der größte Durchmesser des Blockierstifts (2) des Implantats, und der so beschaffen ist, dass er am Körper des Stabilisierungskeils so befestigt werden kann, dass der Kanal parallax zur Achse der Aussparung (12) in dem Körper ist, und
- eine Blockierstift-Einführstange (6), die so beschaffen ist, dass sie in dem inneren Kanal gleitet, um den Blockierstift in der Aussparung des Körpers des Stabilisierungskeils zu platzieren.

9. Chirurgisches Kit nach Anspruch 8, dadurch begrenzt, dass es einem Zwischenwirbelimplantat nach Anspruch 7 entspricht, wobei der röhrenförmige Körper des Implantathalters (5) an einem ersten Ende mit einem Gewinde (51) versehen ist, um mit dem Innengewinde (11) der im Körper des Stabilisierungskeils (1) vorgesehenen Aussparung (12) zusammenzuwirken.

10. Chirurgisches Kit nach einem der Ansprüche 8 oder 9, wobei der Implantathalter an einem zweiten Ende mit einem Innengewinde (53) am Eingang des Kanals (52) versehen ist, und wobei die Einführstange (6) mit einem Gewinde (61) versehen ist, um mit dem Innengewinde zusammenzuwirken, so dass das Einführen und anschließende Einschrauben der Einführstange (6) in den Kanal (52) des Implantathalters (5) den konischen Stift (2) in die im Körper des Keils (1) vorgesehene Aufnahme (12) treibt.

## Claims

1. An intervertebral implant comprising:
- a stabilizing wedge (1) adapted to stabilize at least two adjacent vertebrae by interposition between spinous processes of said vertebrae, having a substantially parallelepipedal body which has a defined main axis,
- at least one strap (4) forming a flexible link for fixing the stabilizing wedge to the spinous processes of the vertebrae to be stabilized, said strap having first and second strap portions (41a, 42a) which each comprise one of the opposite ends (41, 42) of said strap, and said strap having a defined longitudinal direction and a defined strap surface,
- at least one movable assembly (2) adapted to come into engagement with the stabilizing wedge in such a way as to immobilize the strap with respect to the stabilizing wedge by clamping of said strap between said movable assembly and said stabilizing wedge, and
- a locking member (3) for axially locking the engagement of the movable assembly with the stabilizing wedge and thus the blocking of the strap,
***characterized in that***
- the body of the stabilizing wedge comprises at least one recess (12) through which at least the first strap portion can pass, the recess having a longitudinal axis parallel to the main axis of the stabilizing wedge, and an inner wall extending parallel to said longitudinal axis of the recess with a defined shape;
- the movable assembly (2) comprises at least one blocking pin having a defined longitudinal axis and a defined shape substantially complementing the shape of the recess, in order to come into engagement with the stabilizing wedge by movement in said recess in the direction of the main axis of the body of said wedge in such a way that the longitudinal axis of the blocking pin:
- firstly coincides with the longitudinal axis of the recess;
- secondly is perpendicular to the longitudinal axis of the first strap portion inside the recess; and
- thirdly is parallel to the surface of the first strap portion inside the recess,
and moreover in such a way that the strap portion inside the recess is immobilized with respect to the stabilizing wedge by clamping of said strap portion between the blocking pin and the inner wall of the recess.

2. The intervertebral implant as claimed in claim 1, in which:
- each of the first and second strap portions can pass through the recess provided in the stabilizing wedge, in order for the strap to form at least one loop in a plane perpendicular to the main axis of the wedge, with a cord adapted to come into engagement with one of the spinous processes of two vertebrae to be stabilized, and in which furthermore
- the blocking pin can come into engagement with the stabilizing wedge in the direction of the main axis of the body of said wedge, between each of the first and second strap portions (41a, 42a) inside the recess, in such a way that the longitudinal axis of the pin:
- firstly coincides with the longitudinal axis of the recess;
- secondly is perpendicular to the longitudinal axis of each of the two portions of the strap inside the recess; and
- thirdly is parallel to the surface of each of first and second strap portions inside the recess,
and moreover in such a way that each of the first and second strap portions is immobilized with respect to the stabilizing wedge by said strap portion being clamped between the pin and respective portions of the inner wall of the recess that face each other.

3. The intervertebral implant as claimed in claim 2, in which the first and second strap portions can pass in opposite directions through the recess provided in the stabilizing wedge, so as to intersect in said recess, and in order for the strap to form a loop in a plane perpendicular to the main axis of the wedge, with two cords which are located respectively on either side of the stabilizing wedge in said plane and are adapted to come into engagement each with a respective one of the spinous processes of two adjacent vertebrae to be stabilized.

4. The intervertebral implant as claimed in any one of claims 1 through 3, in which the recess provided in the stabilizing wedge has a conical shape, and in which the blocking pin has a conical shape complementing the shape of the recess.

5. The intervertebral implant as claimed in any one of claims 1 through 4, in which the body of the stabilizing wedge comprises passages (13, 14) for the strap (4), which extend perpendicularly with respect to the main axis (10) of the body (1) of said wedge, of which at least one passage passes through the recess (12), and through which passages at least the strap can slide when it is not immobilized with respect to the stabilizing wedge.

6. The intervertebral implant as claimed in any one of claims 1 through 5, in which the strap (4) is a braid made of non-resorbable textile material for medical use.

7. The intervertebral implant as claimed in any one of claims 1 through 6, in which the recess has an internally threaded inlet zone (11) with a diameter larger than the diameter of the recess to the rear of said inlet zone, and in which the locking member is a screw of the same diameter as the diameter of the inlet zone of the recess, with a thread adapted to cooperate with the internal thread of said inlet zone, and with a bearing zone (32) adapted to bear against a contact zone (22) of the blocking pin (2) in the recess (12) when the screw is screwed into the internally threaded inlet zone of the recess.

8. A surgical kit comprising:
- an intervertebral implant as claimed in any one of the preceding claims,
- an implant holder (5) with a tubular body having an an internal channel (52) with an internal diameter slightly larger than the largest diameter of the blocking pin (2) of the implant, and adapted to be fixed to the body of the stabilizing wedge in such a way that the channel is in parallax with the axis of the recess (12) in said body, and
- an insertion rod (6) for the blocking pin, adapted to slide in the internal channel for placing the blocking pin in the recess of the body of the stabilizing wedge.

9. The surgical kit as claimed in claim 8, limited in that it corresponds to an intervertebral implant as claimed in claim 7, in which the tubular body of the implant holder (5) is provided, at a first end, with a thread (51) for cooperating with the internal thread (11) of the recess (12) provided in the body of the stabilizing wedge (1).

10. The surgical kit as claimed in either of claims 8 and 9, in which the implant holder is provided, at a second end, with an internal thread (53) at the inlet of the channel (52), and in which the insertion rod (6) is provided with a thread (61) in order to cooperate with said internal thread in such a way that the insertion and then the screwing of the insertion rod (6) into the channel (52) of the implant holder (5) drives the conical pin (2) inside the recess (12) provided in the body of the wedge (1).
